# EUROPEAN PATENT APPLICATION

(11) **EP 4 481 042 A1**
(43) Date of publication of application: **25.12.2024**
(21) Application number: 23888890.3
(22) Date of filing: 06.09.2023
(51) Int. Cl.: C12N 9/52, C07K 1/18, C07K 1/20, C07K 1/16, C07K 1/34

(54) **METHOD FOR PURIFYING BOTULINUM TOXIN**

(30) Priority: 08.11.2022 KR 20220147467
(71) Applicant: JETEMA CO., LTD., Gangwon-do 26355 (KR)
(72) Inventor: KIM, Jae Young, Seoul 05649 (KR); NAM, Jeong Sun, Seoul 04425 (KR); YUN, Bum Jin, Seoul 05502 (KR); KIM, Seungho, Seoul 03491 (KR); KIM, Minjoong, Suwon-si, Gyeonggi-do 16267 (KR); SHIM, Myungbo, Ansan-si, Gyeonggi-do 15454 (KR); KIM, Jooyeon, Yongin-si, Gyeonggi-do 17074 (KR)
(74) Representative: Crow, Martin
(86) International application number: PCT/KR2023/013358
(87) International publication number: WO 2024/101624

(57) **Abstract**

The present invention relates to a method of purifying botulinum toxin (BTX), wherein the method is performed in the order of purification using cation exchange chromatography (CEX), hydrophobic interaction chromatography (HIC) and mixed mode chromatography (cation exchange (phosphate) and affinity (calcium)), and further includes treating trypsin to activate the botulinum toxin, and as botulinum toxin with excellent purity and activity can be purified, it can be usefully used for botulinum toxin production.

## Description

### [Technical Field]

The present invention relates to a method of purifying botulinum toxin (BTX), and more particularly, to a method of purifying type E botulinum toxin, which is performed in the order of purification using cation exchange chromatography (CEX), hydrophobic interaction chromatography (HIC), and mixed mode chromatography (cation exchange (phosphate) and affinity (calcium)), and further includes an operation of treating trypsin to activate the botulinum toxin.

### [Background Art]

Botulinum toxin is a neurotoxin protein produced by bacteria such as Clostridium butyricum, Clostridium baratii and Clostridium botulinum. Botulinum toxin blocks neuromuscular transmission and causes neuro-paralytic disease in humans and animals. Seven different types of botulinum toxin, A, B, C1, D, E, F, G, and H, have been identified, and each type can be distinguished by each type-specific antibody, and the severity of paralysis they cause and animal species they affect are different.

A molecular weight of the botulinum toxin protein molecule is about 150 kDa, consisting of a heavy chain of about 100 kDa conjugated to a light chain of about 50 kDa. However, botulinum toxin released by Clostridium bacteria is released as a complex between 150 kDa toxin and one or more non-toxin proteins. For example, a botulinum toxin is released as 900 kDa, 500 kDa, and 300 kDa complexes.

Botulinum toxin can be fatal to humans, but recently, botulinum toxin has been developed for the purpose of treating various symptoms including neuromuscular disorders characterized by hyperactivity of skeletal muscles. For example, BOTOX^{®} is a trademark for botulinum toxin A commercially developed by Allergan, Inc., which used for the treatment of blepharospasm, strabismus, cervical dystonia musculorum, and glabella (facial) wrinkles, and other serotypes are being researched to develop suitable uses for them and use them clinically.

Botulinum toxin for clinical use is usually isolated from cell culture, and various purification methods are used.

Botulinum toxin for clinical use is purified in a complexed form by a series of precipitation and tangential flow filtration operations [Schantz E J, et al, Properties and use of botulinum toxin and other microbial neurotoxins in medicine, Microbiol Rev 1992 March 56(1):80-99]. However, this methods provided relatively low yields, typically less than about 10%. Other methods have used size exclusion, ion exchange, and/or affinity chromatography [Schmidt J, et al, Anal Biochem 1986 July; 156(1):213-219; Kannan K, et al, Mov Disord 2000; 15(Suppl 2):20 (2000); Wang YC, Dermatol Las Faci Cosm Surg 2002; 58 (2002); and US Patent No. 2003/0008367].

Another method is to individually synthesize one of the heavy or light chains of botulinum toxin by recombinant means rather than a complete and biologically active botulinum toxin protein [Zhou L, et al, Biochemistry 1995; 34(46):15175-81 (1995); and Johnson S K, et al, Protein Expr and Purif 2003; 32: 1-9 (2003))

More recent methods include use of hydrophobic interaction chromatography, mixed mode chromatography, and/or ion exchange chromatography to purify the botulinum toxin as a complex (US Patent Nos. 7,452,697 and 7,354,740).

However, there is still a need in the art for improved purification methods of isolating stable and biologically active intact botulinum toxin.

Accordingly, the present inventors have made diligent efforts to develop a purification process capable of obtaining type E botulinum toxin with high purity and excellent activity, as a result, it was confirmed that botulinum toxin with very high purity and activity could be purified using processes of cation exchange chromatography, hydrophobic interaction chromatography and mixed mode chromatography, and in particular, the present invention was completed by confirming that botulinum toxin having a purity of 99% or more can be purified when an SP column is used as a cation exchange resin, a butyl column is used as a hydrophobic resin, and a CHT column is used as a mixed mode resin.

### [Disclosure]

### [Technical Problem]

The present invention is directed to providing a method of purifying botulinum toxin (BTX).

### [Technical Solution]

In order to achieve the above object, the present invention provides a method of purifying type E botulinum toxin, including:
(a) pre-treating a culture fluid of a strain producing type E botulinum toxin;
(b) purifying the culture fluid pre-treated in operation (a) using cation exchange chromatography (CEX);
(c) purifying an eluate purified in operation (b) using hydrophobic interaction chromatography (HIC);
(d) activating the type E botulinum toxin by reacting the eluate purified in operation (c) with trypsin; and
(e) purifying a trypsin reaction solution using mixed mode chromatography.

In the present invention, the pre-treating in operation (a) may be ultrafiltration.

In the present invention, the ultrafiltration may be performed using a filtration membrane having a size of 10 kDa to 300 kDa.

In the present invention, the filtration membrane may be a cassette type or hollow fiber type filtration membrane.

In the present invention, the cation exchange chromatography column in operation (b) may be a column packed with a resin including one or more functional groups selected from the group consisting of carboxy methyl (CM), sulfoethyl (SE), sulfopropyl (SP), phosphate (P), and sulfonate (S).

In the present invention, the sulfopropyl (SP) column may be one or more selected from the group consisting of an SP sepharose HP column, an SP sepharose FF column, and a capto S column.

In the present invention, operation (b) may include diluting the culture fluid pre-treated in operation (a) with purified water or a buffer and injecting the diluted culture fluid into the cation exchange chromatography column.

In the present invention, the buffer may be a sodium citrate buffer.

In the present invention, a fraction including the type E botulinum toxin may be obtained by injecting a sodium citrate buffer including sodium chloride as an elution buffer into the column.

In the present invention, the elution buffer may be a10 to 50 mM sodium citrate buffer with a pH of 4.0 to 6.0 to which 0.5 to 1.5 M sodium chloride is added.

In the present invention, the hydrophobic interaction chromatography column in operation (c) may be a column packed with a resin including one or more functional groups selected from the group consisting of ether, isopropyl, butyl, octyl, and phenyl.

In the present invention, a butyl column may be one or more selected from the group consisting of a butyl sepharose HP column, a capto butyl column, a capto phenyl column, and a phenyl HP column.

In the present invention, operation (c) may include diluting the eluate purified in operation (b) in a buffer of pH 4.0 to 6.0 and injecting the diluted eluate into the hydrophobic interaction chromatography column.

In the present invention, the buffer may be a 25 to 75 mM sodium phosphate and/or sodium citrate buffer with a pH of 4.0 to 6.0 to which 3.5 to 4.5 M sodium chloride is added.

In the present invention, a fraction including the type E botulinum toxin may be obtained by injecting a 25 to 75 mM sodium phosphate buffer and/or sodium citrate buffer with a pH of 4.0 to 6.0 into the column.

The present invention may further include, (c') pre-treating the eluate purified in operation (c) after operation (c).

In the present invention, the pre-treating in operation (c') may be diafiltration and/or ultrafiltration.

In the present invention, the diafiltration and/or ultrafiltration may be performed using a filtration membrane having a size of 10 kDa to 300 kDa.

In the present invention, the filtration membrane may be a cassette type or hollow fiber type filtration membrane.

In the present invention, the diafiltration may be performed with a 25 to 75 mM sodium phosphate buffer with a pH of 4.0 to 6.0.

In the present invention, in operation (d), the eluate may be reacted with trypsin at 33 to 36 °C for 0.5 to 2 hours to activate the type E botulinum toxin in the eluate.

In the present invention, the mixed mode chromatography column in operation (e) may be a column (CHT ceramic hydroxyapatite) packed with a resin including a calcium phosphate compound Ca₁₀(PO₄)₆(OH)₂ functional group.

In the present invention, the CHT column may be CHT Type I and/or CHT-XT.

In the present invention, in operation (e), the trypsin reaction solution of operation (d) may be diluted in purified water and injected into a mixed mode chromatography column.

In the present invention, a fraction including the type E botulinum toxin may be obtained by injecting a 5 to 50 mM sodium phosphate buffer with a pH of 4.0 to 6.0 and including 1.5 to 3.0 M sodium chloride into the column.

The present invention also provides type E botulinum toxin prepared by the method.

### [Advantageous Effects]

According to the present invention, type E botulinum toxin having excellent purity and activity can be purified, so that it can be usefully used for production of type E botulinum toxin.

In addition, type E botulinum toxin has the advantage of being effective on the day of injection and lasting for up to one month, so it can be used to manufacture biopharmaceuticals, tissue sealing and facial fixing lifting threads, lipolysis injections, etc.

### [Description of Drawings]

The above and other objects, features and advantages of the present invention will become more apparent to those of ordinary skill in the art by describing exemplary embodiments thereof in detail with reference to the accompanying drawings, in which:
FIG. 1 is a result of analyzing botulinum toxin in an eluate after cation exchange chromatography according to one embodiment of the present invention by FPLC and SDS-PAGE;
FIG. 2 is a result of analyzing botulinum toxin in an eluate after hydrophobic interaction chromatography according to one embodiment of the present invention by FPLC and SDS-PAGE;
FIG. 3 is a result of analyzing a purification operation using trypsin according to one embodiment of the present invention by SDS-PAGE;
FIG. 4 is a result of analyzing botulinum toxin in an eluate after mixed mode chromatography according to one embodiment of the present invention by FPLC and SDS-PAGE;
FIG. 5 is a result of SE-HPLC analysis of the purity of botulinum toxin purified according to the present invention;
FIG. 6 is a result of analyzing samples for each operation of botulinum toxin purification by SDS-PAGE according to the present invention;
FIG. 7 is a result of RP-HPLC analysis of the purity of botulinum toxin purified according to the present invention;
FIG. 8 is a result of confirming the HPLC purity of botulinum toxin purified under optimized conditions; and
FIG. 9 is a comparison result of SDS-PAGE for a botulinum toxin cleaving ability according to trypsin-derived species.

### [Modes of the Invention]

Hereinafter, the present invention will be described in more detail.

Unless defined otherwise, all technical and scientific terms used herein have the same meaning as commonly understood by one of ordinary skill in the art to which this invention belongs. In general, the nomenclature used herein is well known and commonly used in the art.

In the present invention, the term "botulinum toxin" refers not only to a neurotoxin produced by Clostridium botulinum, but also to botulinum toxin (or a light chain or a heavy chain thereof) recombinantly produced by non-Clostridium-based species. As used herein, botulinum toxin also includes both pure botulinum toxin (for example, about 150 kDa) as well as botulinum toxin complexes (for example, 300, 600 and 900 kDa complexes). The "pure botulinum toxin" is defined as botulinum toxin that is isolated or substantially isolated from other proteins, including proteins that form the botulinum toxin complex. The pure botulinum toxin may have a purity of 95% or more, preferably 99% or more.

In the present invention, the strain producing botulinum toxin may be Clostridium botulinum or a variant thereof, most preferably a Clostridium botulinum type E, but is not limited thereto, and it will be apparent to those skilled in the art that any strain capable of producing botulinum toxin can be used.

Therefore, in one aspect, the present invention relates to a method of purifying type E botulinum toxin, including.
(a) pre-treating a culture fluid of a strain producing type E botulinum toxin;
(b) purifying the culture fluid pre-treated in operation (a) using cation exchange chromatography (CEX);
(c) purifying an eluate purified in operation (b) using hydrophobic interaction chromatography (HIC);
(d) activating type E botulinum toxin by reacting the eluate purified in operation (c) with trypsin; and
(e) purifying a trypsin reaction solution using mixed mode chromatography.

In the present invention, the pre-treating in operation (a) may be ultrafiltration, but is not limited thereto.

In the present invention, the ultrafiltration may be performed using a filtration membrane having a size of 10 kDa to 300 kDa, preferably 50 kDa to 100 kDa, but is not limited thereto.

In the present invention, the filtration membrane may be a cassette type or hollow fiber type filtration membrane, but is not limited thereto.

In the present invention, the cation exchange chromatography column in operation (b) may be a column packed with a resin including one or more functional groups selected from the group consisting of carboxy methyl (CM), sulfoethyl (SE), sulfopropyl (SP), phosphate (P), and sulfonate (S), but is not limited thereto.

In particular, a sulfopropyl (SP) column can effectively remove impurities in the cation exchange chromatography and maintain high activity of the botulinum toxin. The sulfopropyl (SP) column may include an SP sepharose HP column, an SP sepharose FF column, a capto S column, or the like, and the SP sepharose HP column may be preferably used, but is not limited thereto.

In one embodiment of the present invention, the SP sepharose HP column includes a sulfopropyl functional group and may be in the form of a 6% spherical, cross-linked agarose matrix, DBC is 55 mg/mL based on Ribonuclease A, and may be a column packed with a resin having a particle size of 34 µm, but is not limited thereto.

In the present invention, in the case of purification using a type of chromatography (for example, anion exchange chromatography, hydrophobic chromatography, or mixed mode chromatography) other than cation exchange chromatography in operation (b) and a column thereof, the purification yield and purity of type E botulinum toxin may be lowered compared to the case of purification using the cation exchange chromatography and a column thereof (for example, a sulfopropyl (SP) column) of the present invention.

In the present invention, operation (b) may include diluting the culture fluid pre-treated in operation (a) with purified water or a buffer and injecting the diluted culture fluid into the cation exchange chromatography column, but is not limited thereto.

In the present invention, the concentration and pH of the buffer may be suitable for binding type E botulinum toxin to the cation exchange chromatography column, but is not limited thereto.

In the present invention, the buffer may be a sodium citrate buffer, but is not limited thereto.

In the present invention, a fraction including type E botulinum toxin can be obtained by injecting a sodium citrate buffer including sodium chloride as an elution buffer into the column, but is not limited thereto.

In the present invention, the elution buffer may be a 10 to 50 mM sodium citrate buffer with a pH of 4.0 to 6.0 to which 0.5 to 1.5 M sodium chloride is added, but is not limited thereto.

In operation (b) of the present invention, impurities are removed, and type E botulinum toxin in complex form can be captured.

In the present invention, the hydrophobic interaction chromatography column in operation (c) may be a column packed with a resin including one or more functional groups selected from the group consisting of ether, isopropyl, butyl, octyl, and phenyl, but is not limited thereto.

In particular, a butyl column can effectively separate proteins in non-complexed form. The butyl column may include a butyl sepharose HP column, a capto butyl column, a capto phenyl column, a phenyl HP column, and the like, and the butyl sepharose HP column may be preferably used, but is not limited thereto.

In one embodiment of the present invention, the butyl sepharose HP column has a highly cross-linked agarose 6% matrix, exhibits a degree of substitution of 50 µmol butyl groups/mL, and may be a column packed with resin with a bead size of 34 µm, but is not limited thereto.

In the present invention, in the case of purification using a type of chromatography (for example, cation exchange chromatography, anion exchange chromatography, or mixed mode chromatography) other than hydrophobic interaction chromatography in operation (c) and a column thereof, the purification yield and purity of type E botulinum toxin may be lowered compared to the case of purification using the hydrophobic interaction chromatography and a column thereof (for example, a butyl column) of the present invention.

In the present invention, operation (c) may include diluting the eluate purified in operation (b) in a buffer of pH 4.0 to 6.0 and injecting the diluted eluate into the hydrophobic interaction chromatography column, but is not limited thereto.

In the present invention, the buffer may be a 25 to 75 mM sodium phosphate and/or sodium citrate buffer with a pH of 4.0 to 6.0 to which 3.5 to 4.5 M sodium chloride is added, but is not limited thereto.

In the present invention, the concentration and pH of the buffer may be suitable for binding type E botulinum toxin to the hydrophobic interaction chromatography column, but is not limited thereto.

In the present invention, a fraction including type E botulinum toxin can be obtained by injecting a 25 to 75 mM sodium phosphate and/or sodium citrate buffer with a pH of 4.0 to 6.0 into the column, but is not limited thereto.

Impurities (75 kDa) may be additionally removed in operation (c) of the present invention, but is not limited thereto.

The present invention may further include, (c') pre-treating the eluate purified in operation (c) after operation (c), but is not limited thereto.

In the present invention, the pre-treating in operation (c') can be performed by diafiltration and/or ultrafiltration, but is not limited thereto.

In the present invention, the filtration membrane may be a cassette type or hollow fiber type filtration membrane, but is not limited thereto.

In the present invention, the diafiltration and/or ultrafiltration may be performed using a filtration membrane having a size of 10 kDa to 300 kDa, preferably 30 kDa to 50 kDa, but is not limited thereto.

In the present invention, the diafiltration may be performed with a 25 to 75 mM sodium phosphate buffer with a pH of 4.0 to 6.0, but is not limited thereto.

In operation (d) of the present invention, when trypsin is treated at 33 to 36 °C for 0.5 to 2 hours, a peptide bond (covalent bond) between a heavy chain (HC, 100 kDa) and a light chain (LC, 50 kDa) of 150 kDa of type E botulinum toxin protein may be cleaved to activate botulinum toxin.

That is, in the present invention, in operation (d), the eluate is reacted with trypsin at 33 to 36 °C for 0.5 to 2 hours to activate type E botulinum toxin in the eluate, but is not limited thereto.

In the present invention, the trypsin may be porcine-derived trypsin, but is not limited thereto.

In the present invention, the mixed mode chromatography column in operation (e) may be a column (CHT ceramic hydroxyapatite) packed with a resin including a calcium phosphate compound Ca₁₀(PO₄)₆(OH)₂ functional group, but is not limited thereto.

The CHT column can effectively remove other impurities and increase the purity of ~ 300 kDa botulinum toxin. The CHT column may use CHT Type I, CHT-XT, and the like, and preferably, the CHT-XT column may be used, but is not limited thereto.

In the present invention, in the case of purification using a type of chromatography (for example, cation exchange chromatography, anion exchange chromatography or hydrophobic interaction chromatography) other than mixed mode chromatography in operation (e) and a column thereof, the purification yield and purity of type E botulinum toxin may be lowered compared to the case of purification using the mixed mode chromatography and a column thereof (for example, a CHT column) of the present invention.

In the present invention, in operation (e), the trypsin reaction solution of operation (d) may be diluted in a purified water and injected into a mixed mode chromatography column, but is not limited thereto.

In the present invention, the dilution with purified water may lower the buffer concentration of a trypsin reaction solution, so that type E botulinum toxin may bind to the mixed mode chromatography column, but is not limited thereto.

In the present invention, a fraction including type E botulinum toxin can be obtained by injecting a 5 to 50 mM sodium phosphate buffer with a pH of 4.0 to 6.0 and including 1.5 to 3.0 M sodium chloride into the column, but is not limited thereto.

In operation (e) of the present invention, impurities (75 kDa) are further removed, and the purity of the botulinum toxin complex can be increased.

In the present invention, the mixed mode chromatography operation may use any mixed mode chromatography process known in the art. The mixed mode chromatography includes the use of solid phase chromatography supports in the form of resins, monoliths or membranes that use multiple chemical mechanisms to adsorb proteins or other solutes. Examples useful in the present invention include, but are not limited to, chromatographic supports using a combination of two or more of the following mechanisms: anion exchange, cation exchange, hydrophobic interaction, hydrophilic interaction, thiophilic interaction, hydrogen bonding, pi-pi bonding, and metal affinity. In certain embodiments, mixed mode chromatography processes combine (1) anion exchange and hydrophobic interaction techniques; (2) cation exchange and hydrophobic interaction techniques; and/or (3) electrostatic and hydrophobic interaction techniques. In one embodiment, the mixed mode chromatography operation can be accomplished by using columns and resins such as Capto^{®} adhere columns and resins available from GE Healthcare Life Sciences. The Capto^{®} adhere column is a multimodal medium for intermediate purification and polishing of monoclonal antibodies after capture. In certain embodiments, the mixed mode chromatography operation can be performed in a flow-through mode. In another aspect, the mixed mode chromatography operation can be performed in a bind-elute mode. In another aspect, the mixed mode chromatography operation can be accomplished using one or more of the following systems: Capto^{®} MMC(Cytiva), HEA HyperCel^{™} (Pall Corporation), PPA HyperCel^{™}(Pall Corporation), MBI HyperCel^{™}(PallCorporation), MEP HyperCel^{™}(PallCorporation), Blue Trisacryl M(Pall Corporation), CFT^{™} Ceramic Fluoroapatite(Bio-Rad Laboratories, Inc.), CHT^{™} Ceramic Hydroxyapatite(Bio-Rad Laboratories, Inc.) and/or ABx^{™}(J.T. Baker). Particular methods used for the mixed mode chromatography operation may depend on the particular column and resin used, and are typically supplied by the manufacturer or known in the art.

In one embodiment of the present invention, the cation exchange chromatography column in operation (b) is a sulfopropyl (SP) column, the hydrophobic interaction chromatography column in operation (c) is a butyl column, and in operation (e), the mixed mode chromatography column may be a CHT-XT column, but is not limited thereto, but when a different type of column is used, the purification yield and purity of type E botulinum toxin may be lowered.

The botulinum toxin purified by the method may be type E botulinum toxin with a purity of 99% or more, and the purified botulinum toxin may have a higher purity than botulinum toxin purified by an existing method.

Accordingly, in another aspect, the present invention relates to type E botulinum toxin prepared by the method.

As used herein, the term "fraction" refers to a group in which at least one target molecule (for example, botulinum toxin) included in a biopharmaceutical preparation, together with one or more impurities, passes through a substance that binds to one or more impurities, and in a separation method in which the target molecule does not usually bind (that is, flow-through) or is eluted after binding, materials that have passed through, including the target molecule, are separated and collected.

The term "purification" used in the present invention refers to an operation to increase purity by removing mixed impurities from a substance, and in the present specification, refers to a process in which purification is to separate the botulinum toxin produced as botulinum bacteria overgrow and then die from a culture fluid of the botulinum bacteria, and is used as a method to improve purity during a botulinum toxin production process.

Hereinafter, the present invention will be described in more detail through examples. These examples are only for illustrating the present invention, and it will be apparent to those of ordinary skill in the art that the scope of the present invention is not to be construed as being limited by these examples.

### [Examples]

### Example 1

### Pre-treatment process (ultrafiltration) of culture fluid of type E botulinum toxin-producing strain

A TFF System was equipped with a Pellicon^{®}2 Mini Cassette (50 kDa, 0.1 m2). A recovered culture fluid volume, pH, and conductivity were measured. A recovered culture fluid was primarily concentrated through ultrafiltration. Purified water was added to a primary concentration process liquid, and secondary concentration was performed to recover a secondary concentration process liquid. Purified water was added to the TFF system, and the remaining process liquid was recovered and combined with the already recovered secondary concentrated process liquid. The combined final process liquid amount, pH, and conductivity were confirmed.

### Example 2

### Purification process of type E botulinum toxin

### (Example 2-1) Cation exchange chromatography

A column packed with SP-HP resin was equipped with a fast protein liquid chromatography (FPLC) apparatus. A final process liquid prepared in Example 1 was adjusted to pH 5.0 using 1 N hydrogen chloride (HCl), and the process liquid was injected into the column. After injection, the column was washed with an equilibration/wash buffer (20 mM sodium citrate, pH 5.0). After washing, an equilibration/elution buffer (20 mM sodium citrate, 1 M sodium chloride, pH 5.0) was injected, and fractions in the 0 to 0.5 M sodium chloride section were sequentially obtained. Each of the fractions was confirmed by SDS-PAGE, and samples for SDS-PAGE analysis were prepared under the conditions shown in Table 1.

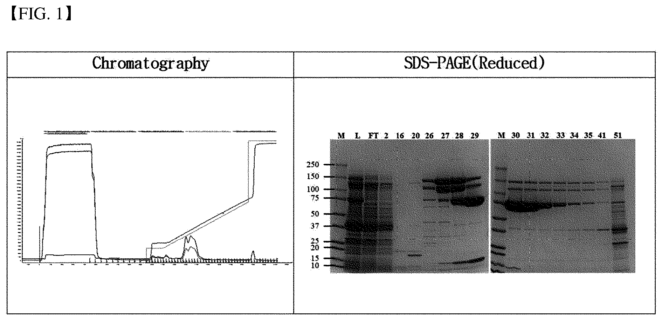

As a result of confirming each of the fractions by SDS-PAGE, it was confirmed that botulinum toxin in a form of a complex of ~300 kDa was purified from the fraction (FIG. 1).

**[Table 1]**

| **Sample preparation conditions for SDS-PAGE analysis** | | |
|---|---|---|
| **Components** | **Reduced condition** | **Non-reduced condition** |
| Botulinum toxin(1 mg/mL) | 3 uL | 3 uL |
| LDS Sample buffer(4x) | 5 uL | 5 uL |
| Sample reducing agent(10x) | 2 uL | 0 uL |
| Purified water | 10 uL | 12 uL |
| Total volume | 20 uL | |

### (Example 2-2) Hydrophobic interaction chromatography

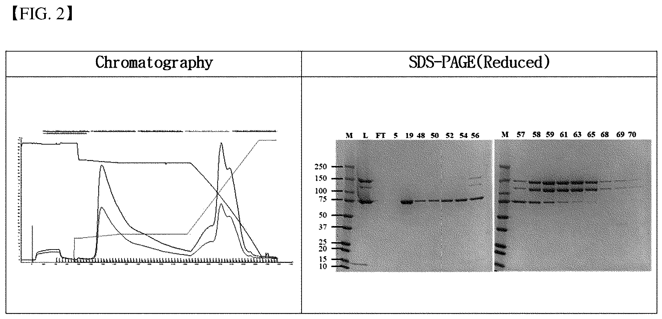

A column packed with a butyl-HP resin was equipped with a fast protein liquid chromatography (FPLC) apparatus. A fraction eluted from the SP-HP column of Example 2-1 was titrated to pH 5.0 with 1 N sodium hydroxide. A 50 mM sodium citrate buffer with a pH of 5.0 and including 4 M sodium chloride was added to the SP-HP process eluate so that conductivity became 170 to 180 mS/cm. The eluate to which the buffer was added was injected into the column. After injection, the column was washed with an equilibration/wash buffer (50 mM sodium citrate, 2.5 M sodium chloride, pH 5.0). After washing, an equilibration/elution buffer (50 mM sodium citrate, pH 5.0) was injected, and fractions in the 0 to 2.5 M sodium chloride section were sequentially obtained (FIG. 2).

As a result of confirming each of the fractions by SDS-PAGE, it was confirmed that a large amount of impurities (75 kDa) were removed, and botulinum toxin in a form of a complex of ~300 kDa was purified from the fraction (FIG. 2).

### (Example 2-3) Ultrafiltration and diafiltration (UF/DF)

A TFF System was equipped with Sartocon Slice 200 (30 kDa, 0.02 m2). An amount of eluate eluted from the butyl-HP column was confirmed and pH and conductivity were measured. An eluate obtained in secondary hydrophobic interaction chromatography was subjected to at least 1,000-fold diafiltration (DF) with 50 mM sodium phosphate, pH 5.8 (hereafter referred to as DF buffer). The UV absorbance, pH, and conductivity of a final process liquid were measured. The final process liquid was filtered through a 0.2 µm filter and stored at 4 °C.

### (Example 2-4) Trypsin treatment

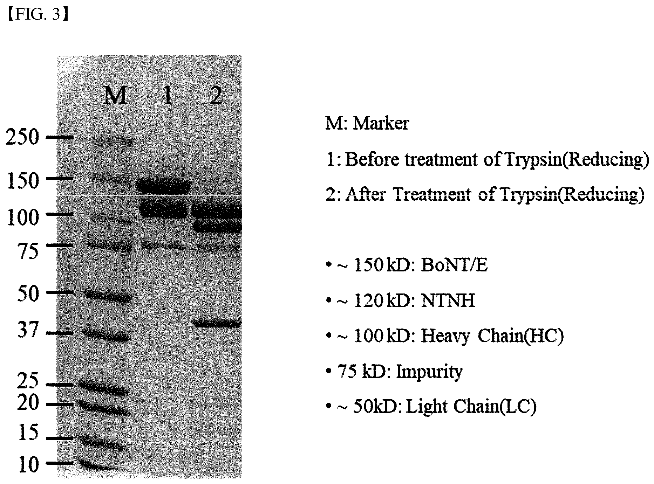

After reacting the final process liquid of Example 2-3 with trypsin (Roche, 06369880) at 35 °C for 1 hour, the trypsin reaction solution was recovered. The trypsin reaction solution was diluted 1:4 using cooled purified water. A diluted reaction solution amount, pH, and conductivity were confirmed. After confirming pH, titration was performed to pH 5.8 using 1 N sodium hydroxide, and each fraction was confirmed by SDS-PAGE (FIG. 3).

As a result, it was confirmed that density of a 150 kDa neurotoxin protein (Neurotoxin) was 5% or more and was divided into a heavy chain (HC, 100 kDa) and a light chain (LC, 50 kDa) (FIG. 3).

### (Example 2-5) Mixed mode chromatography

A column packed with CHT-XT (resin) was equipped with a fast protein liquid chromatography (FPLC) apparatus. The trypsin reaction solution of Example 2-4 was injected into a column. After injection, the column was washed with an equilibration/wash buffer (10 mM sodium phosphate, pH 5.8). After washing, an equilibration/elution buffer (10 mM sodium phosphate, 2 M sodium chloride, pH 5.8) was injected, and fractions in the 0 to 2.0 M sodium chloride section were sequentially obtained.

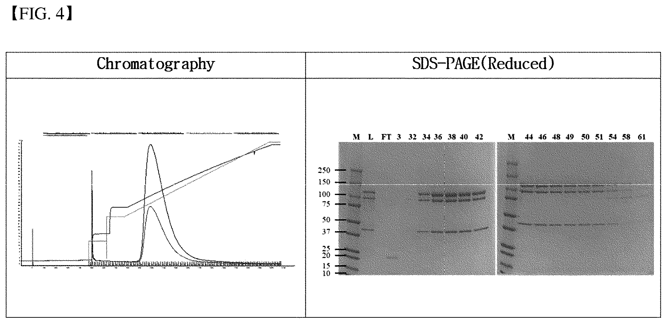

As a result of confirming each of the fractions by SDS-PAGE, it was confirmed that a large amount of impurities (75 kDa) were removed, and botulinum toxin in a form of a complex of ~300 kDa was purified from the fraction (FIG. 4).

### Example 3

### Comparison of type E botulinum toxin SDS-PAGE for each purification operation

Fractions including the botulinum toxin purified in Example 2 were collected and, and as a commercially available botulinum toxin research reference standard, Clostridium botulinum type E complex toxin (Metabiologics, Inc. Lot: E010821-01) product was used, the product was diluted with a buffer solution (10 mM sodium phosphate, pH 5.8) to a concentration of 1 mg/mL, and divided into reduced conditions and non-reduced conditions as shown in Table 1, and samples were prepared for SDS-PAGE. The samples were electrophoresed in Novex Wedge Well 8 to 16% Tris-Glycine, 10 wells (Invitrogen, XP08160BOX), and after about 30 mL of InstantBlue reagent was added, put on a shaker and stained for 60 minutes, then the staining reagent was completely removed, and after about 30 mL of purified water was added, a process of washing on the shaker for 30 minutes was repeated at least 5 times. When the staining was sufficiently removed and band confirmation was possible, the gel was analyzed using an Image Analyzer.

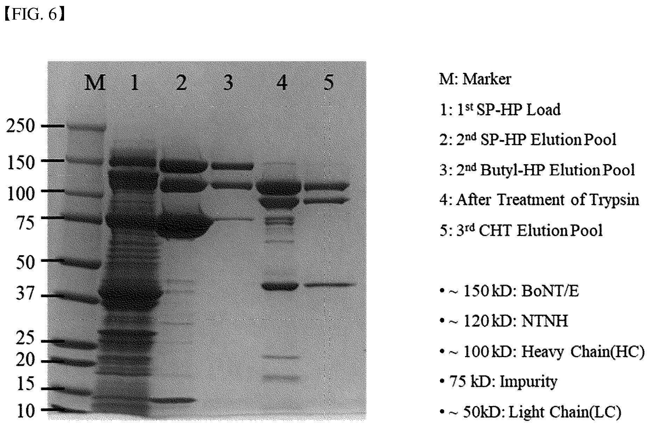

As a result, a purification process of a neurotoxin protein for each operation was confirmed from SDS-PAGE (FIG. 6), and the yield at each operation was confirmed (Table 2). It was found that the purification method of the present invention can purify only the correct neurotoxin protein.

**[Table 2]**

| **Toxin protein yield for each purification operation** | | |
|---|---|---|
| **Sample Name** | **Amount (mg)** | **Yield (%)** |
| Culture medium | 105.20 | 100.00 |
| SP-HP | 84.90 | 80.70 |
| Butyl-HP | 52.80 | 50.19 |
| CHT-XT | 35.90 | 34.13 |

### Example 4

### Activity of purified botulinum toxin product of the present invention

Using the botulinum toxin purified in Example 3, a specific titer value of the stock solution was evaluated through a titer test using animals. Mortality (LD50) was measured for 3 days after intraperitoneal injection of 0.1 mL to 10 female mice weighing 18 to 22 g per group. The number of deaths measured for 3 days was calculated as a specific titer by probit analysis using CombiStats, a statistical program. The test was repeated three times, and an average specific titer value of the three repetitions was evaluated as 4.5 × 10⁷ U/mg.

The results are shown in Table 3, and it was confirmed that the purification method according to the present invention can purify the botulinum toxin without weakening its activity.

**[Table 3]**

| ***Activity of purified botulinum toxin product*** | |
|---|---|
| **Test** | **Potency(U/mg)** |
| Test 1 | 4.5 X 10⁷ |
| Test 2 | 4.4 X 10⁷ |
| Test 3 | 4.6 X 10⁷ |
| **Average** | **4.5** X **10⁷** |

### Example 5

### Confirmation of purification process purity

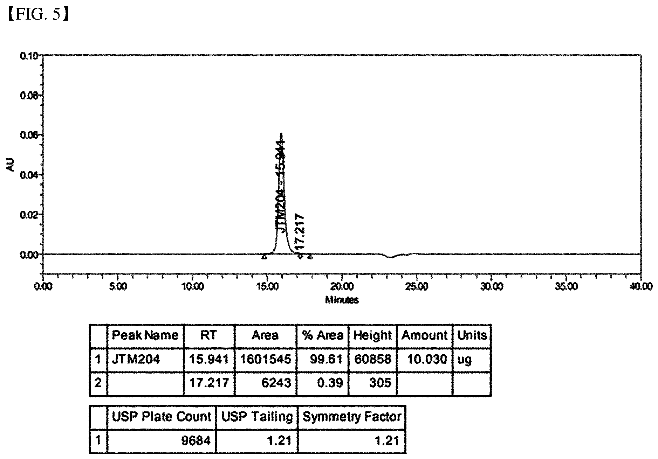

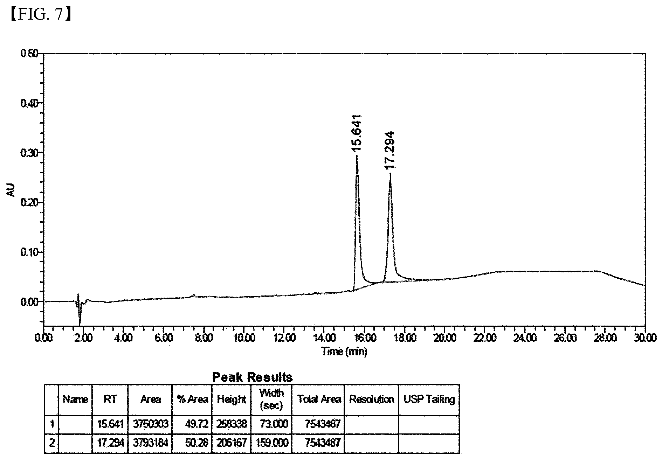

Purified botulinum toxin was analyzed for purity by SE-HPLC and RP-HPLC using the conditions in Tables 4 and 5. SE-HPLC results are shown in FIG. 5, and it was confirmed that the purity was 99.61% at a retention time (RT) of 15.9 min. In addition, RP-HPLC results are shown in FIG. 7, two main peaks were confirmed and no impurity peak was confirmed. A peak at a retention time of 15.6 min was confirmed as pure botulinum toxin, and the peak at a retention time of 17.2 min was confirmed as NTNH of a complex component.

**[Table 4]**

| ***SE-HPLC analysis conditions*** | |
|---|---|
| **Item** | **SE-HPLC Condition** |
| Column | PROTEIN LW-803 |
| Size | 8 mm X 300 mm |
| Stationary phase | Silica gel for chromatography R (3 µm) |
| Column temperature | 25 ± 5 °C |
| Sampler temperature | 5 ± 3 °C |
| Mobile phase | 100mM Citric acid-Na2PO4, pH 5.6, 0.3 M NaCl |
| Flow rate | 0.5 mL/min |
| Running time | 30 min |
| Detection | UV detector, 278 nm |
| Injection volume | 10 µL (10 µg) |

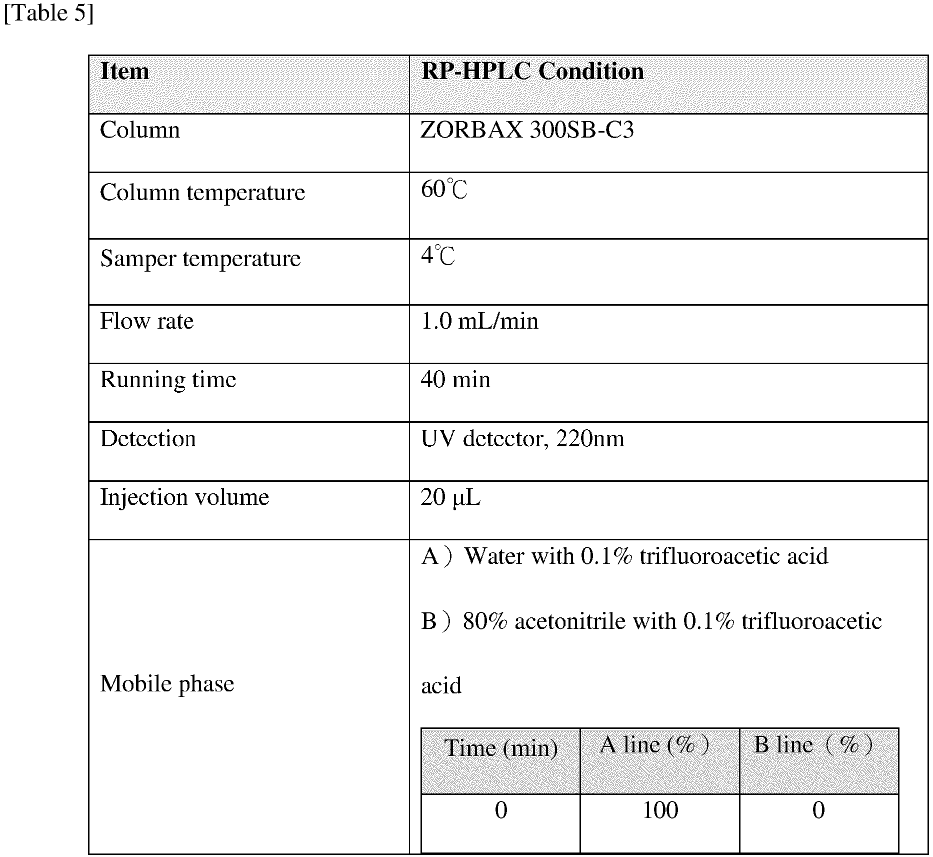

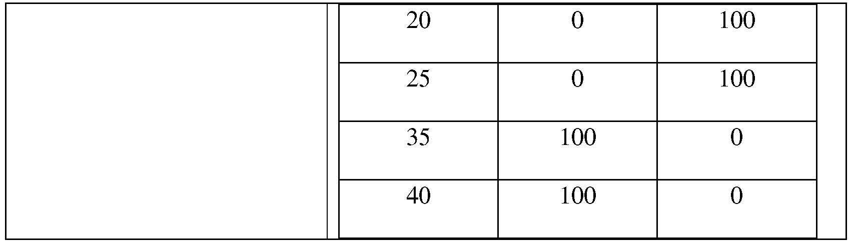

### Example 6

### In Process Control (IPC) analysis result of purified botulinum toxin

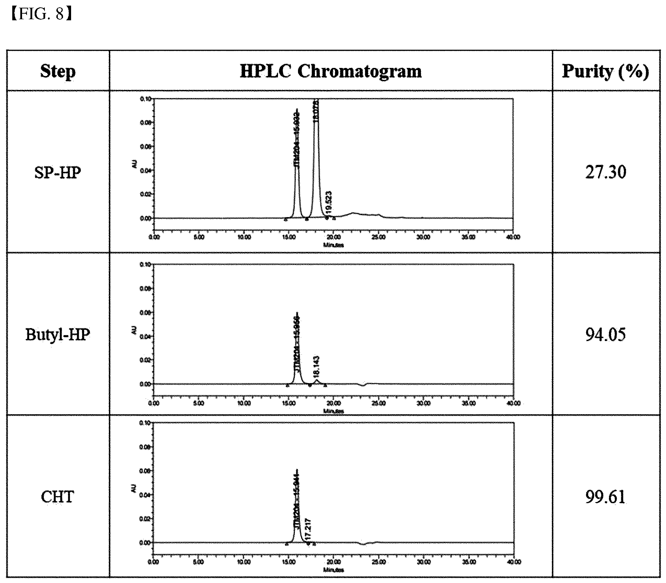

According to the results of Example 2, the product purified by a primary SP-HP column, a secondary butyl-HP column, and a tertiary CHT-XT column was analyzed by HPLC to determine purity (Table 4). As a result, it was confirmed that purity of the botulinum toxin was 99.61% (FIGS. 5 and 8).

### Example 7

### Comparison of botulinum toxin cleavage ability according to trypsin-derived species

It is known that the use of animal-derived trypsin other than bovine-derived trypsin is not effective for activating and purifying botulinum toxin. Thus, the present inventors compared and analyzed a case of using porcine-derived trypsin and a case of using bovine-derived trypsin (Sigma, T1426). Specifically, after reacting porcine-derived trypsin and bovine-derived trypsin at a temperature of 33 to 36 °C for 0.5 to 2 hours, a trypsin inhibitor was added (the same ratio as the amount of trypsin added to botulinum toxin) to stop the enzymatic reaction, and the sample was analyzed by SDS-PAGE.

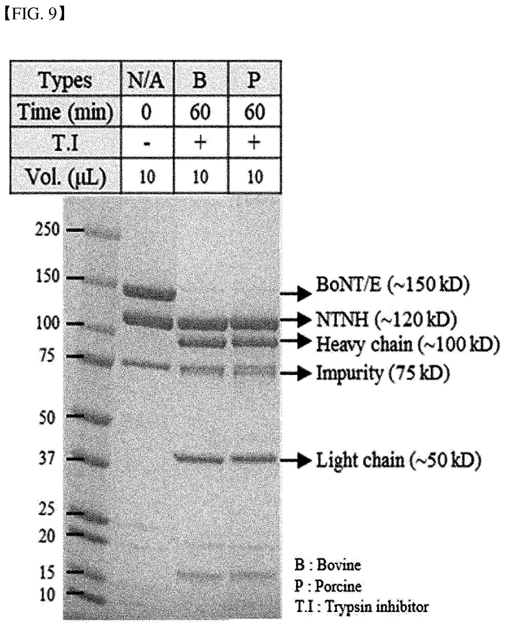

As a result, it was confirmed that there was no significant difference between the case of using the porcine-derived trypsin and the case of using the bovine-derived trypsin in comparison of a botulinum toxin cleavage ability under the above conditions (FIG. 9).

## Claims

1. A method of purifying type E botulinum toxin, comprising:
(a) pre-treating a culture fluid of a strain producing type E botulinum toxin;
(b) purifying the culture fluid pre-treated in operation (a) using cation exchange chromatography (CEX);
(c) purifying an eluate purified in operation (b) using hydrophobic interaction chromatography (HIC);
(d) activating the type E botulinum toxin by reacting the eluate purified in operation (c) with trypsin; and
(e) purifying a trypsin reaction solution using mixed mode chromatography.

2. The method of claim 1, wherein the pre-treating in operation (a) is ultrafiltration.

3. The method of claim 2, wherein the ultrafiltration is performed using a filtration membrane having a size of 10 kDa to 300 kDa.

4. The method of claim 3, wherein the filtration membrane is a cassette type or hollow fiber type filtration membrane.

5. The method of claim 1, wherein a cation exchange chromatography column in operation (b) is a column packed with a resin including one or more functional groups selected from the group consisting of carboxy methyl (CM), sulfoethyl (SE), sulfopropyl (SP), phosphate (P), and sulfonate (S).

6. The method of claim 5, wherein a sulfopropyl (SP) column is one or more selected from the group consisting of an SP sepharose HP column, an SP sepharose FF column, and a capto S column.

7. The method of claim 1, wherein operation (b) comprises diluting the culture fluid pre-treated in operation (a) with purified water or a buffer and injecting the diluted culture fluid into a cation exchange chromatography column.

8. The method of claim 7, wherein the buffer is a sodium citrate buffer.

9. The method of claim 7, wherein a fraction including the type E botulinum toxin is obtained by injecting a sodium citrate buffer including sodium chloride as an elution buffer into the column.

10. The method of claim 9, wherein the elution buffer is a 10 to 50 mM sodium citrate buffer with a pH of 4.0 to 6.0 to which 0.5 to 1.5 M sodium chloride is added.

11. The method of claim 1, wherein a hydrophobic interaction chromatography column in operation (c) is a column packed with a resin including one or more functional groups selected from the group consisting of ether, isopropyl, butyl, octyl, and phenyl.

12. The method of claim 11, wherein a butyl column is one or more selected from the group consisting of a butyl sepharose HP column, a capto butyl column, a capto phenyl column, and a phenyl HP column.

13. The method of claim 1, wherein operation (c) comprises diluting the eluate purified in operation (b) in a buffer of pH 4.0 to 6.0 and injecting the diluted buffer into a hydrophobic interaction chromatography column.

14. The method of claim 13, wherein the buffer is a 25 to 75 mM sodium phosphate and/or sodium citrate buffer with a pH of 4.0 to 6.0 to which 3.5 to 4.5 M sodium chloride is added.

15. The method of claim 13, wherein a fraction including the type E botulinum toxin is obtained by injecting a 25 to 75 mM sodium phosphate buffer and/or sodium citrate buffer with a pH of 4.0 to 6.0 into the column.

16. The method of claim 1, further comprising (c') pre-treating the eluate purified in operation (c) after operation (c).

17. The method of claim 16, wherein the pre-treating in operation (c') is performed by diafiltration and/or ultrafiltration.

18. The method of claim 17, wherein the diafiltration and/or ultrafiltration is performed using a filtration membrane having a size of 10 kDa to 300 kDa.

19. The method of claim 18, wherein the filtration membrane is a cassette type or hollow fiber type filtration membrane.

20. The method of claim 17, wherein the diafiltration is performed with a 5 to 50 mM sodium phosphate buffer with a pH of 4.0 to 6.0.

21. The method of claim 1, wherein in operation (d), the eluate is reacted with trypsin at 33 to 36 °C for 0.5 to 2 hours to activate the type E botulinum toxin in the eluate.

22. The method of claim 1, wherein a mixed mode chromatography column in operation (e) is a column (CHT ceramic hydroxyapatite) packed with a resin including a calcium phosphate compound Ca₁₀(PO₄)₆(OH)₂ functional group.

23. The method of claim 22, wherein the CHT column is CHT Type I and/or CHT-XT.

24. The method of claim 1, wherein in operation (e), the trypsin reaction solution of operation (d) is diluted in purified water and injected into a mixed mode chromatography column.

25. The method of claim 24, wherein a fraction including the type E botulinum toxin is obtained by injecting a 5 to 50 mM sodium phosphate buffer with a pH of 4.0 to 6.0 and including 1.5 to 3.0 M of sodium chloride into the column.

26. Type E botulinum toxin prepared by the method of any one of claims 1 to 25.
